# EUROPEAN PATENT APPLICATION

(11) **EP 1 229 012 A1**
(43) Date of publication of application: **07.08.2002**
(21) Application number: 00971459.3
(22) Date of filing: 27.10.2000
(51) Int. Cl.: C07C 41/50, C07C 43/303, B01J 29/00

(54) **PROCESS FOR PREPARING BETA-KETOESTER ACETAL**

(30) Priority: 28.10.1999 ES 9902439
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, Avelino, E-46022 Valencia (ES); VELTY, Alexandra, Isabelle, Lucienne, E-46022 Valencia (ES); CLIMENT OLMEDO, Maria José, E-46022 Valencia (ES); SUSARTE ROGEL, Manuel, E-46022 Valencia (ES)
(74) Representative: Ungria Lopez, Javier
(86) International application number: ES0000415
(87) International publication number: WO0130733

(57) **Abstract**

The present invention provides a process for the preparation of a β-keto ester acetal by acetalization of a β-keto ester with alkyl glycol, characterized in that the acetalization is carried out in the presence of an acid catalyst selected among the group comprised of acid forms of zeolites, zeotypes, mesoporous molecular sieves with a pore size between 20 and 100Å, amorphous silicas with a pore size between 20 and 150Å, silicoaluminophosphates and mesoporous silicates with a pore size between 20 and 150Å by means of which high yields and selectivities are obtained. Another object of the present invention is a β-keto ester acetal obtained according to the process described herein.

## Description

### FIELD OF THE INVENTION

The present invention fits in the area of chemistry and in particular it refers to a process for the preparation of a β-keto ester acetal.

### BACRGROUND OF THE INVENTION

Protection of carbonyl groups in the form of acetals is many times an indispensable requirement in the synthesis of polyfunctional organic molecules, that are of great interest in Fine Chemistry. Furthermore, there is a series of dimethylacetals and cyclic acetals widely used as aromas in perfumery as well as in the food and pharmaceutical industries. Among them we can point out phenylacetaldehyde derivatives (phenylacetaldehyde dimethylacetal and 2-methyl-3-phenylacetaldehyde) and ethyl acetoacetate ethylenedioxyacetal (POMAL or FRUCTONA), of cedrene, etc.

The formation of acetals is normally carried out by homogeneous catalysis, by treating the carbonyl compound with an excess of the corresponding alcohol or glycol under acid catalysis, eliminating the water formed in the reaction by physical methods (continuous elimination by azeotropic distillation with an inert solvent) or chemicals (using orthoesters, dehydrating agents, etc.). The acids most used in acetalization have been hydrochloric acid, sulfuric acid and p-toluenesulfonic acid (F.A.J. Meskens, *Synthesis* (1981), 501).

POMAL is a compound commonly used in the food industry and in perfumery (Ford, R.A., Leticia, C. *Food.Chem. Toxicol.* 1988, 26(4), 327), that has a smell similar to that of an apple.

The preparation of POMAL (ethyl 3-ethylenedioxy butanoate) is generally carried out in a homogeneous phase and in the presence of conventional acid catalysts such as sulfuric acid or p-toluenesulfonic acid.

Catalysts with a marked acid force like the two above-mentioned ones, normally used in acetalization with homogeneous catalysis have the inconvenience that in the presence of the water formed as a reaction product they cause hydrolysis of the ester giving rise in the case of acetalization of POMAL, for example, of 3-ethylenedioxy butanoic acid. The presence of small amounts of this compound considerably alters the organoleptic characteristics of the final product.

F. A. J. describes in Synthesis (Reviews), (1981) 508, the formation of FRUCTONA using HCl acid (g) as the catalyst. The yield after 3 h of reaction is 58%.

Over the last decades, the introduction of heterogeneous catalysts in the petrochemical industry has created a great interest in the use of this type of material in different chemical processes. The advantages that these materials offer include among others: easy handling, acid neutralization steps are avoided, selectivity of form, possibility to reuse the catalyst and furthermore, the absence of contaminating wastes.

The heterogeneous catalysts used include alumina (T.W. Greene, R.G.M. Wuts, *In Protective groups in Organic Synthesis,* 2^{nd}. Ed., Wiley, N.Y., (1991), Chapter 4 pp. 175; Vu Thuy, Maitte P., *Bull. Soc. Chim. Fr.* (1975) 9, clays (K. Baver, P. Garbe, H. Surburg, *Common Fragrance and Flavor Materials. Preparation properties and uses,* 2^{nd}. Ed. 1990, VCH Publishers, N.Y.), zirconium oxide (Shibagaki, Makoto, K. Hideyuki, T. Kyoko, M. Hajime, Japanese patent JP63146838, 1986), zeolites (A. Corma, M.J. Climent, H. Garcia and J. Primo. *Appl. Catal.* 59 (1990) 333, M.J. Climent, A. Corma, S. Iborra, M.C. Navarro and J. Primo. *J. Catal.* 161 (1996) 783; M.V. Joshi and C.S. Narasimhan, *J. Catal.,* 128 (1991) and R. Ballini, G. Bosica, B. Frullanti, R. Maggi, G. Sartori, F. Schroer, *Tetrahedron Lett.* 39 (1998) 1615 montmorillonite (Tong-Shuang Li, Sheng-Hui Li, Ji-Tai Li and Hui-Zhang Li, *J. Chem. Research (S),* (1997) 26).

Wang, Cunde et al. in *Huaxue Shijie*, 34(1) (1993) 20-2, carry out acetalization of different carbonyl compounds (among them ethyl acetoacetate) with ethylene glycol and propylene glycol in the presence of zeolites (Y) exchanged with Sn⁺², Zn⁺², Ca⁺², Ni⁺² and NH₄⁺. The reaction is carried out using benzene as a solvent at 103°C obtaining yields of the different cyclic acetals in the range of 99%.

Xu, Peng-Fi et al. describe in *Hecheng Huaxue,* 3(4) (1995) 363-6 the formation of cyclic acetals using ethylene glycol and different carbonyl compounds among which is ethyl acetoacetate in the presence of ZrCl₄ or TiCl₄. The yield of POMAL obtained by means of this process after 16 h of reaction is 72%.

Wang, Cunde et al. in Xiandai Huagong 16(12), (1996) 25-6 obtain ethyl 3-ethylenedioxy butanoate with a yield of 90% after 1.5 hours of reaction from a mixture of ethyl acetoacetate and ethylene glycol at the reflux temperature of the mixture in the presence of aluminum sulfate.

Jiang, Wei et al. describe in *Chin. Chem. Lett.* 8(5), (1997) 377-80 the acetalization of different carbonyl compounds with ethylene glycol, in the presence of zeolite HY. The formation of POMAL is carried out using two zeolites HY₁ (Si/Al = 5) and HY₂ (Si/Al = 9.5) in a percentage by weight of 31% with respect to the starting acetoacetate, obtaining yields of 83% and 100%, respectively.

Tong-Shuang Li et al. in *J. Chem. Research (S)* (1997) 26-7 describe the formation of ethyl acetoacetate ethylenedioxy upon heating a mixture of ethyl acetoacetate and ethylene glycol in the presence of montmorillonite K-10 using benzene as the solvent. In these conditions the yield of the corresponding acetal after 6 hours of reaction is 98%.

Wang, Cunde et al. in *Lizi Jiaohuan Yu Xifu,* 14(2) (1998) 156-159 carry out acetalization in the presence of resins exchanged with ions obtaining good yields. Ethyl acetoacetate is cited among the carbonyl compounds and it is not indicated that alcohol or polyol is used.

The specific case of the preparation of POMAL would be another typical example in which the use of acid catalysts, and more specifically zeolites, as catalysts, not only have the advantages associated with the above-cited heterogeneous catalysts, but by using zeolites it is also possible to control the acid strength of the catalyst, as well as the hydrophilic-hydrophobic characteristics of the surface thereof, thus controlling the adsorption of the products. The optimization of these factors permits the most selective catalyst to be chosen in order to obtain the desired product.

### OBJECT OF THE INVENTION

The object of the present invention is to overcome the inconveniences of the above-mentioned prior art, providing a process for the preparation of β-keto ester acetals, using an alkyl glycol as a starting product and in the presence of acid heterogeneous catalysts.

Another object of the present invention is a β-keto ester acetal obtained by the process described herein.

### DETAILED DESCRIPTION OF THE INVENTION

The present invention provides a process for the preparation of a β-keto ester acetal by means of the acetalization of a β-keto ester with alkyl glycol, characterized in that the acetalization is carried out in the presence of an acid catalyst selected among the group comprised of acid forms of zeolites, zeotypes, mesoporous molecular sieves with a pore size between 20 and 100Å, amorphous silicas with a pore size between 20 and 150 Å, silicoaluminophosphates and mesoporous silicates with a pore size between 20 and 150 Å.

The process can be carried out in a stirred tank type continuous or discontinuous reactor, or in a continuous fixed or fluidized bed reactor, wherein the catalyst is located. In the event a stirred tank type reaction system is used, the reactor can have a built-in Dean-Stark system that permits the water that is produced during the course of the reaction to be displaced and/or a system that permits the process to be performed at pressures lower than 10⁵ Pa.

The process of the present invention is preferably discontinuous.

The catalyst is used in a proportion between 0.01% and 30% by weight, as referred to the β-keto ester. In a preferred embodiment the catalyst is used in a proportion between 0.1% and 10% by weight, as referred to the β-keto ester.

Said catalyst can be selected among zeolites, zeotypes and mesoporous silicates with Si/T^{III} ratios in the lattice comprised in the range of 6 and 400, wherein T^{III} refers to at least a trivalent element. In a preferred embodiment the Si/T^{III} ratios in the lattice are comprised in a range between 10 and 200. In an even more preferred embodiment said Si/T^{III} ratios in the lattice are comprised in a range of 25 and 50, wherein T^{III} refers to at least a trivalent element.

The catalyst may preferably be a zeolite that has pores formed by rings with at least 10 members. Among the zeolites that can be used as catalysts is the group comprised of zeolites Beta (BEA), ITQ-7, faucenite (Y), ZSM-5, Mordenite, Omega, NU-86, NU-87, SSZ-33, MFI, SSZ-24 (AFI), SSZ-26, MCM-22, ITQ-2, Ofretite (OFF), ZSM-12 (AFI) and SSZ-42, ultrastable USY, USY (the abbreviations between parenthesis are the ones accepted by the International Zeolite Association (W.M. Meier and D.H. Olson in Atlas of Zeolites Structure Types, 1992) ultrastable zeolites exchanged with elements selected among divalent and trivalent elements and combinations of divalent and trivalent elements, new zeolites that combine channels of 10 MR with 14 MR such as CIT-5 and UTD-1, ultrastable faujasites, ultrastable faujasites exchanged with elements selected among divalent and trivalent elements and combinations of divalent and trivalent elements, as well as isomorphic substitutions thereof with trivalent elements other than aluminum.

A group of zeolites especially suitable for the process of the invention are three-directional large pore zeolites.

Zeolites Beta and zeolites ITQ-7 are especially preferred among said large pore zeolites. Large pore three-directional zeolites Beta-1 and ITQ-7 are especially suitable for the obtainment of POMAL obtaining good yields and selectivities with them.

The catalyst used in the present process can have a Si/Al ratio in the lattice between 25 and 50. Zeolites Beta whose Al content has been modified during the synthesis thereof are especially suitable to achieve Si/Al ratios in the lattice comprised between this range. Said zeolites Beta have the best properties of adsorption and acidity for the obtainment of POMAL, obtaining with them good yields and selectivity. In the absence of an inert solvent three-directional zeolites are still the most appropriate ones in order to obtain POMAL, whereas medium pore zeolites (ZSM-5) and mono-directional large pore zeolites (mordenite) have diffusion problems.

SAPO-5 and SAPO-37 can be used among silicoaluminophosphate type catalysts.

The catalyst may also be a silicate of mesoporous materials with a uniform distribution of pores, such as for example, M41S, HMS, KIT and SAM.

Among mesoporous molecular sieves with a pore size between 20 and 100Å, that may act as catalysts are for example MCM-41, MCM-48, HMS, KIT and amorphous silica aluminas with a pore size in a narrow range of porosities (Bellusi et al. *Stud. Surf. Sci.* 84, 93 (1994).

The present process can be carried out at a temperature between 25 and 280°C for 2 minutes to 50 hours and with a β-keto ester:alkyl glycol ratio between 0.5:1 and 1:10.

In a preferred embodiment the process is carried out at a temperature between 25 and 280°C, for a period of time between 10 minutes and 12 h, and with a β-keto ester:alkyl glycol molar ratio between 1:1 and 1:2.

A β-keto ester with a general formula of R₁COCH₂COOR₂ wherein R₁ and R₂ groups are the same or different and are selected among linear alkyl groups, branched alkyl groups, cycloalkyl groups, phenyl groups, naphthyl groups and heterocycles with a number of carbon atoms between 1 and 25 is used as a starting product in the present process.

R₁ and R₂ groups may be selected between methyl and ethyl.

The β-keto ester may preferably be ethyl acetoacetate.

According to the process of the present invention, the alkyl glycol may have a number of carbon atoms between 2 and 12 and may be selected among primary, secondary, linear, branched, saturated and unsaturated polyols. Preferably the polyol is selected among saturated primary alkyl glycols. Likewise, the alkyl glycol used preferably has a number of carbon atoms between 2 and 6. Even more preferably said polyol is selected between ethylene glycol and propylene glycol.

In accordance with the present process an inert solvent selected among aliphatic hydrocarbons, aromatic hydrocarbons, halogenated hydrocarbons, ethers, nitriles such as acetonitrile, tetrahydrofuran and mixtures thereof may be used. For example, pentane, cyclohexane, petroleum ether, decane may be used among aliphatic hydrocarbons. For example, benzene, toluene, xylene may be used among aromatic hydrocarbons. Some examples of suitable solvents among halogenated hydrocarbons are carbon tetrachloride, methylene chloride or chloroform. Some examples among ethers are diethyl ether, diisopropyl ether, dioxane, anisole or tetrahydrofuran.

According to the present process when the inert solvent is used, a system like the Dean-Stark system is used to displace the water formed during the reaction.

As the final step of the process, the catalyst used is washed, for example, with dichloromethane and the solvent is evaporated. The catalyst is available to be used again.

The process may be carried out in an alternative embodiment in the absence of a solvent under atmospheric pressure, without eliminating the water formed with Dean-Stark equipment, but rather continuously distilling the water formed a d/or by means of a vacuum distillation system. In the event that the process is carried out in the absence of a solvent, the process is performed at a pressure lower than 10⁵ Pa. The pressure of the system is preferably kept lower than 1.013x10⁵ Pa, preferably between 133.322x10² Pa and 666.61 Pa. The temperature range used in the event that the process is carried out according to this alternative way, in the absence of a solvent, is between 25 and 100°C, preferably at 40°C. As in the case of the embodiment of the process with a solvent, the last step consists of recovering the catalyst, by means of filtering, washing with a solvent in order to recover the catalyst and evaporation of the solvent in order to obtain the final product.

In a preferred embodiment of the process a phenylacetaldehyde derivative acetal is prepared. In an even more preferred embodiment said acetal is an acetal selected between phenylacetaldehyde dimethylacetal and 2-methyl-3-phenyl phenylacetaldehyde dimethylacetal. Said phenylacetalaldehyde derivative acetal is preferably prepared with continuous extraction of the water formed, in the presence of toluene as a solvent. Said phenylacetaldehyde derivative acetal can also be prepared with extraction of the water formed by applying a pressure lower than 1.013x10⁵ and a temperature between 40 and 250°C.

In another preferred embodiment of the process the acetal that is obtained is ethyl ethylenedioxyacetal.

In another preferred embodiment of the process the acetal that is obtained is cedrene acetal.

In another preferred embodiment of the process ethyl acetoacetate is acetylated with ethylene glycol in the presence of an acid catalyst selected among acid forms of zeolites Beta with a Si/Al ratio in the lattice between 10 and 50, ZSM-5 with a Si/Al ratio in the lattice between 10 and 100, Mordenite with a Si/Al ratio in the lattice between 10 and 50, USY with a Si/Al ratio in the lattice between 5 and 100 and ITQ-7 with a Si/Al ratio in the lattice between 25 and 50, at a molar ethyl acetoacetate:ethylene glycol ratio between 1:1 and 1:2 and at a temperature between 25 and 280°C and for a reaction time between 10 minutes and 12 hours.

A discontinuous process is preferably used in the acetylation of ethyl acetoacetate with ethylene glycol and furthermore the catalyst is used in a proportion of 0.01% to 30% by weight as referred to the ethyl acetoacetate; even more preferably a catalyst is used in a proportion between 0.1% and 10% by weight referred to the ethyl acetatoacetate. Said acid catalyst is preferably selected among zeolites Beta with a Si/Al ratio of 13, zeolites Beta with a Si/Al ratio of 15, zeolites Beta with a Si/Al ratio of 25 and zeolites Beta with a Si/Al ratio of 50. The ethyl acetoacetate may also preferably be acetylated with ethylene glycol with continuous extraction of the water formed in the presence of toluene as the solvent.

The ethyl acetoacetate acetal may also be obtained without a solvent, with extraction of the water formed by evaporation by applying a pressure lower than 1.013x10⁵ Pa and at a temperature between 40 and 250°C.

Before the catalysts are used, they should be prepared in their acid form.

### Preparation of the acid form:

The preparation of the acid form of the catalyst in the specific case that the catalyst is a zeolite is described hereinafter; but this method may apply to the rest. In the event that it is a zeolite, said acid form is prepared, either directly by exchange with a mineral acid, when the stability of the zeolite permits this, either by an indirect method consisting of replacing the alkaline and alkaline earth cations thereof by NH₄⁺ by treatment with an aqueous solution of ammonium chloride followed by calcination of the product obtained by conventional methods. It may also be prepared by exchange with divalent or trivalent ions, followed by thermal treatment at temperatures between 100 and 600 °C, for periods between 30 minutes and 6 hours.

On the other hand, parameters such as the number of acid centers, the distribution of acid strength and the hydrophilic-hydrophobic characteristics of the catalyst can be controlled by varying the Si/Al proportion of the crystalline lattice. The Al content of a zeolite may be directly modified in the synthesis step, and when this is not possible post-synthesis dealuminizations that mainly consist of extracting aluminum from the lattice by treatment with steam with acid or with complexing agents such as for example EDTA are used. If so desired, silicon may be introduced at the same time by means of chemical treatment with reagents such as (NH₄)₂F₆Si or SiCl₄.

The advantages provided by the process of the present invention are mainly derived from the use of heterogeneous catalysts and among others they consist of easy handling, which avoids neutralization steps of the acid used in conventional homogenous catalysis, selectivity of the form, the possibility to reuse the catalyst and furthermore the lack of contaminating wastes.

Another object of the present invention is β-keto ester acetal, obtained according to the process described.

Various examples the purpose of which is to illustrate some aspects of the invention are described hereinafter.

### EXAMPLE 1

### Acetalization of ethyl acetoacetate in the presence of zeolites with different crystalline structures

Immediately before its use, the catalyst in an amount equivalent to 7.4% by weight with respect to the starting ethyl acetoacetate is activated by heating to 100°C under pressure of 133.32 Pa for 2 h. After this time the system is allowed to cool to room temperature and a mixture of 12 mmoles (1.5 g) of ethyl acetoacetate and 24 mmoles (1.5g) of ethylene glycol, dissolved in 40 ml. of dry toluene is added to it. The system is connected to Dean-Stark equipment for azeotropic distillation of water. The suspension is magnetically stirred at a constant temperature of 146°C. After 2h of reaction, the zeolite is washed and filtered with dichloromethane. The resulting solution is washed with water in order to eliminate the excess ethylene glycol and then the solvents are eliminated in a rotary evaporator. The crude of the reaction is analyzed by gas chromatography - mass spectrometry (GC-MS) and H-NMR. The yields of POMAL (**1**) and of 3-ethylenedioxy butanoic acid (**2**) expressed in percentages by mass (%) after 1h of reaction for the different crystalline structures are the following:

| Catalyst (Si/Al) | Yield **1**(%) | Yield **2**(%) | Selectivity **1** |
|---|---|---|---|
| Beta-1 (13) | 91 | 4 | 96 |
| ZSM-5 (40) | 93 | 2 | 95 |
| Mordenite (10) | 84 | 2 | 95 |
| ITQ-2 (25) | 70 | 4 | 92 |
| MCM-41 (15) | 50 | 15 | 77 |

### EXAMPLE 2

### Acetalization of ethyl acetoacetate in the presence of zeolites Beta (hydrophobic) with a different Si/Al ratio

In the same conditions as in example 1 but using 0.6% of catalyst, the reaction is carried out with four samples of zeolite Beta with Si/Al ratios of 15, 25, 50 and 100 (prepared as described in the patent of S. Valencia, Camblor M.A. and Corma A., S.P. P9600625 (1996)). The yields of cyclic acetal **1** and of acid **2** after 1 hour of reaction are the following:

| Catalyst (Si/Al) | Yield **1**(%) | Yield **2**(%) | Selectivity **1** |
|---|---|---|---|
| Beta-2 (15) | 51 | 8 | 86 |
| Beta-3 (25) | 93 | 1 | 99 |
| Beta-4 (50) | 82 | 3 | 96 |
| Beta-5 (100) | 11 | <1 | 97 |

### EXAMPLE 3

### Acetalization of ethyl acetoacetate in the presence of Beta-3 modifying the volume of the solvent

The acetalization reaction was carried out in the same way as in example 1 but using 4% of zeolite Beta-3 and modifying the volume of toluene. The results of 1 obtained after 1 h of reaction as well as the selectivity are found in the following table:

| V_{Toluene}/V_{AAEt} | Yield **1**(%) | Yield **2**(%) | Selectivity 1 |
|---|---|---|---|
| 26.4 | 81 | 1 | 98 |
| 16.4 | 95 | 1 | 97 |
| 8.3 | 98 | <1 | 97 |
| 5.5 | 98 | <1 | 97 |
| 2.2 | 99 | <1 | 98 |

### EXAMPLE 4

### Acetalization in the absence of inert solvents at reduced pressure in the presence of zeolites with different crystalline structures

A solution of ethyl acetoacetate (6 g, 46 mmol) and ethylene glycol (5.7 g, 92 mmol) is added to a flask where the previously activated catalyst is located. The amount of catalyst used is 5% with respect to the starting acetoacetate. The system is connected to a vacuum equipment, which maintains a constant pressure of 1066.56 Pa. The resulting suspension is kept under stirring in a silicone bath at the temperature of 40°C. After 2 hours of reaction the catalyst is filtered and washed with dichloromethane. The results obtained are in the following table and in all cases the selectivity of POMAL is 100%.

| Catalyst (Si/Al) | Yield **1** (%) |
|---|---|
| Beta-1 (13) | 97 |
| Beta-3 (25) | 95 |
| Mordenite (10) | 44 |
| MCM-41 (15) | 8 |

## Claims

1. A process for the preparation of a β-keto ester acetal by means of acetalization of a β-keto ester with alkyl glycol, **characterized in that** the acetalization is carried out in the presence of an acid catalyst selected among the group comprised of acid forms of zeolites, zeotypes, mesoporous molecular sieves with a pore size between 20 and 100Å, amorphous silicas with a pore size between 20 and 150Å, silicoaluminaphosphates and mesoporous silicates with a pore size between 20 and 150 Å.

2. A process according to claim 1, **characterized in that** it is a discontinuous process and **in that** a catalyst is used in a proportion between 0.01% and 30% by weight, as referred to the β-keto ester.

3. A process according to claim 2, **characterized in that** the catalyst is used in a proportion between 0.1% and 10% by weight, as referred to β-keto ester.

4. A process according to any of claims 1 to 3, **characterized in that** the catalyst is selected among zeolites, zeotypes and mesoporous silicates with Si/T^{III} ratios in the lattice comprised in a range between 6 and 400, wherein T^{III} refers to at least a trivalent element.

5. A process according to any of claims 1 to 3, **characterized in that** the catalyst is selected among zeolites, zeotypes and mesoporous silicates with Si/T^{III} ratios in the lattice comprised in a range of 10 and 200, wherein T^{III} refers to at least a trivalent element.

6. A process according to any of claims 1 to 3, **characterized in that** the catalyst is selected among zeolites, zeotypes and mesoporous silicates with Si/T^{III} ratios in the lattice in a range between 25 and 50, wherein T^{III} refers to at least a trivalent element.

7. A process according to any of claims 1 to 6, **characterized in that** the catalyst is selected among zeolites and with pores formed by rings of at least 10 members.

8. A process according to any of the preceding claims, **characterized in that** the catalyst is selected among the group comprised of zeolites Beta, ITQ-7 Y, ZSM-5, Mordenite, Omega, NU-86, NU-87, SSZ-33, MFI, SSZ-24, SSZ-26, MCM-22, ITQ-2, SSZ-24, OFF, ZSM-12 and SSZ-42, CIT-5, UTD-1, ultrastable USY, ultrastable USY exchanged with elements selected among divalent and trivalent elements and combinations of divalent and trivalent elements, ultrastable faujasites, ultrastable faujasites exchanged with elements selected among divalent and trivalent elements and combinations of divalent and trivalent elements, as well as isomorphic substitutions thereof with trivalent elements other than aluminum.

9. A process according to any of the preceding claims, **characterized in that** the catalyst is selected among large pore three-directional zeolites.

10. A process according to claim 1 or 9, **characterized in that** the catalyst is selected among zeolites Beta and zeolites ITQ-7.

11. A process according to claim 10, **characterized in that** the catalyst has a Si/Al ratio in the lattice between 25 and 50.

12. A process according to claim 1, **characterized in that** the catalyst is a silicoaluminophosphate selected between SAPO-5 and SAPO-37.

13. A process according to any of claims 1 to 5, **characterized in that** the catalyst is a silicate of mesoporous materials with a uniform distribution of pores selected among M41S, HMS, KIT and SAM.

14. A process according to any of the claims 1 to 13, **characterized in that** it is carried out at a temperature between 25 and 280°C for 2 minutes to 50 hours and with a β-keto ester:alkyl glycol molar ratio between 0.5:1 and 1:10.

15. A process according to any of the claims 1 to 14, **characterized in that** it is carried out at a temperature between 25 and 280°C for 10 minutes to 12 hours and with a β-keto ester:alkyl glycol molar ratio between 1:1 and 1:2.

16. A process according to any of claims 1 to 15, **characterized in that** it uses a β-keto ester of general formula R₁COCH₂COOR₂ wherein the R₁ and R₂ groups are the same or different and they are selected among linear alkyl groups, branched alkyl groups, cycloalkyl groups, phenyl groups, naphthyl groups and heterocycles, with a number of carbon atoms between 1 and 25.

17. A process according to claim 16, **characterized in that** R₁ and R₂ are selected between methyl and ethyl.

18. A process according to claim 17, **characterized in that** the β-keto ester is ethyl acetoacetate.

19. A process according to any of claims 1 to 18, **characterized in that** an alkyl glycol with a number of carbon atoms between 2 and 12 and selected among primary, secondary, linear, branched, saturated and unsaturated polyols is used.

20. A process according to claim 19, **characterized in that** the polyol is selected among saturated primary alkyl glycols.

21. A process according to claim 19 or 20, **characterized in that** the alkyl glycol has a number of carbon atoms between 2 and 6.

22. A process according to claim 19 or 20, **characterized in that** the polyol is selected between ethylene glycol and propylene glycol.

23. A process according to any of claims 1 to 22, **characterized in that** it uses an inert solvent selected among aliphatic hydrocarbons, aromatic hydrocarbons, halogenated hydrocarbons, ethers, nitriles, tetrahydrofuran and mixtures thereof.

24. A process according to claim 23, **characterized in that** the inert solvent is selected among pentane, cyclohexane, petroleum ether, decane, benzene, toluene, xylene, carbon tetrachloride, methylene chloride, chloroform, diethyl ether, diisopropyl ether, dioxane, anisole, acetonitrile, tetrahydrofuran and mixtures thereof.

25. A process according to claim 22, 23 or 24, **characterized in that** when the inert solvent is used, a system that displaces the water formed (Dean-Stark system) is used.

26. A process according to any of claims 1 to 23, **characterized in that** it is carried out in the absence of a solvent at atmospheric pressure, without eliminating the water formed with a Dean-Stark equipment.

27. A process according to any of claims 1 to 23 or 25, **characterized in that** it is carried out in the absence of a solvent at a pressure lower than 1 bar, continuously distilling the water formed.

28. A process according to any of claims 1 to 16 or 28 to 27, **characterized in that** an acetal of a phenylacetaldehyde derivative is prepared.

29. A process according to claim 28, **characterized in that** the acetal is phenylacetaldehyde dimethylacetal.

30. A process according to claim 28, **characterized in that** the acetal is 2-methyl-3-phenyl phenylacetaldehyde.

31. A process according to claim 28, **characterized in that** the acetal is ethyl ethylenedioxyacetal.

32. A process according to claim 28, **characterized in that** the acetal is cedrene acetal.

33. A process according to claim 28, **characterized in that** ethyl acetoacetate is acetylated with ethylene glycol
in the presence of an acid catalyst selected among acid forms of zeolites Beta with a Si/Al ratio in the lattice between 10 and 50, ZSM-5 with a Si/Al ratio in the lattice between 10 and 100, Mordenite with a Si/Al ratio in the lattice between 10 and 50, USY with a Si/Al ratio in the lattice between 5 and 100 and ITQ-7 with a Si/Al ratio in the lattice between 25 and 50
at an ethyl acetoacetate:ethylene glycol molar ratio between 1:1 and 1:2, and
at a temperature between 25 and 280°C for a reaction time between 10 minutes and 12 hours.

34. A process according to claim 33, **characterized in that** it is discontinuous and **in that** the catalyst is used in a proportion of 0.01% to 30% by weight as referred to the ethyl acetoacetate.

35. A process according to claim 33 or 34, **characterized in that** the acid catalyst is selected among zeolites Beta with a Si/Al ratio of 13, zeolites Beta with a Si/Al ratio of 15, zeolites Beta with a Si/Al ratio of 25 and zeolites Beta with a Si/Al ratio of 50.

36. A process according to claim 28, 34 or 35, **characterized in that** the water formed is continuously extracted in the presence of toluene as a solvent.

37. A process according to claim 28, 34 or 35, **characterized in that** it is carried out without a solvent with the water formed extracted by evaporation by applying a pressure lower than 1.013x10⁵ Pa and at a temperature between 40 and 250°C.

38. A β-keto ester acetal obtained according to any of claims 1 to 37.
